# EUROPEAN PATENT APPLICATION

(11) **EP 2 149 371 A1**
(43) Date of publication of application: **03.02.2010**
(21) Application number: 08161229.3
(22) Date of filing: 28.07.2008
(51) Int. Cl.: A61K 31/18, A61P 15/06

(54) **Use of steroid sulfatase inhibitors for the treatment of preterm labor**

(71) Applicant: PregLem S.A., 1228 Plan-les-Ouates (CH)
(72) Inventor: Loumaye, Ernest, F-74140 Massongy (FR); Cayron-Elizondo, Valérie, F-74160 Collonges-sous-Saleve (FR); Gotteland, Jean-Pierre, F-74160 Beaumont (FR)
(74) Representative: reuteler & cie SA

(57) **Abstract**

The present invention is related to a use of a steroid sulfatase inhibitor in the manufacture of a medicament for preventing or inhibiting premature uterine contractions. Specifically, the present invention is related to steroid sulfatase inhibitors useful for the preparation of a pharmaceutical formulation for the modulation, notably the inhibition of pre-term labor.

## Description

### Field of the Invention

The present invention relates to a use of a steroid sulfatase inhibitor in the manufacture of a medicament for preventing or inhibiting premature uterine contraction. The present invention further relates to a method for preventing or treating premature uterine contractions. Specifically, the present invention is related to sulfamate steroid sulfatase inhibitors useful for the preparation of a pharmaceutical formulation for preventing or treating pre-term labor.

### Background of the Invention

Preterm delivery is a significant health problem *(*Slattery et al., Lancet, 2002, 360(9344), 1489-97*).* Although preterm birth may be caused deliberately in either the mother or baby's interest, up to 50% preterm births result from idiopathic preterm labour, which may or may not be preceded by spontaneous membrane rupture *(*Barros et al., 2006, Obstet. Gynecol., 107(5):1035-41*).* Given that preterm birth is the major cause of perinatal death, and that the costs associated with caring for infants born preterm are immense (estimated in the USA at $ 26.2 billion in total or over $ 50,000 dollars per infant born preterm) *(Medicine lo., 2007, Institute of Medicine, 398-429).*

The physiology of human parturition involves "ripening" of the uterine cervix (the process by which it changes from a firm rigid structure which is able to hold the baby in the uterus, to a soft, more compliant structure which can be passively opened by contractions of the myometrium) and the initiation of myometrial contractions. Once the cervix has been opened by myometrial contractions, the foetus is expelled by myometrial contractions aided by maternal effort. Preterm labour appears to involve a similar process, hence therapeutic strategies to prevent preterm birth have focused on prevention of initiation of myometrial contractions (e.g. prophylactic progesterone therapy), inhibition of myometrial contractions after they have become clinically apparent (tocolysis), prevention of premature cervical opening (usually using a suture: cervical cerclage) or strategies to prevent the adverse sequelae associated with foetal membrane rupture (antibiotic therapy). Although these treatments are of some assistance in reducing the risk of preterm birth, none have been shown conclusively to improve perinatal mortality or morbidity. Indeed, the most effective "therapy" currently available for the management of preterm birth is administration of glucocorticoids to improve foetal lung maturation prior to birth: although this has no effect on the likelihood of preterm delivery it reduces each of perinatal mortality, respiratory distress syndrome and intraventricular haemorrhage, with typical odds ratios of 0.45- 0.6 in babies treated *in utero* compared to those not treated.

In many animals such as the sheep and the goat, the timing of parturition is regulated by the foetal pituitary-adrenal axis. Rising levels of glucocorticoids, produced by the foetal adrenal gland in response to rising foetal ACTH, cause an increase in the oestrogen /progesterone ratio which leads directly to stimulation of factors (such as prostaglandin F2α) causing uterine contractions and hence parturition. The crucial role of the adrenal gland in these animals is demonstrated by studies showing that foetal injections of ACTH induce parturition, and that foetal hypophysectomy or adrenalectomy delay parturition indefinitely.

The control of human parturition is less clear. Current theories suggest that the initiation of parturition results from a parturition "cascade" in which factors maintaining uterine quiescence (such as progesterone, prostacyclin, nitric oxide and relaxin) are removed, and those promoting uterine contractions (such as estrogens, prostaglandins, oxytocin and connexin 43, the major protein of myometrial gap junctions) are increased *(*Norwitz et al., 1999, The New England Journal of Medicine, 341, 660-666*).* Although the foetal adrenal gland appears important in human parturition, its role as a key orchestrator is less clear given that parturition can occur relatively normally when either the foetal pituitary gland is absent (anencephaly) or the foetal adrenal glands are hypoplastic. Additionally, an increase in oestrogen/progesterone serum levels have not been consistently demonstrated in human parturition.

Preterm birth may arise from the premature initiation of normal physiological processes, but may also be triggered by intrauterine infection and/or inflammation.

Steroid sulfatase or steryl sulfatase (STS) is a microsomal enzyme catalyzing the hydrolysis of aryl and alkyl steroid sulfates *(*Reed et al., 2005, Endocr. Rev., 26(2), 171-202*)* which has an essential role in regulating the formation of biologically active steroids. It is notably crucial for the local production of active estrogens and androgens through the conversion of their systemic circulating sulphate precursors, namely oestrone sulphate (E1S) and dehydroepiandrosterone sulphate (DHEAS), respectively. Both estrone and dehydroepiandrosterone can be converted to steroids with estrogenic properties (i.e estradiol and androstenediol).

STS has been cloned, expressed *(*Stein et al., 1987, J. Biol. Chem., 264:13865-13872*;* Yen et al., 1987, Cell, 49: 443-454*)* and allocated the enzyme number EC 3.1.6.2. STS has been identified as being implicated in a number of disease conditions. It has been found that a total deficiency in STS produces ichthyosis. According to some workers, STS deficiency is fairly prevalent in Japan. *Sakura et al. (*Sakura et al., 1997, J Inherit. Metab. Dis., 20(6):807-10*)* have also reported that allergic diseases - such as bronchial asthma, allergic rhinitis, or atopic dermatitis - may be associated with a steroid sulfatase deficiency. In addition to disease states being brought on through a total lack of STS activity, an increased level of STS activity may also bring about disease conditions. By way of example, there is strong evidence to support a role of STS in breast cancer growth and metastasis. Therefore, STS inhibitors (STS-I) are currently developed for the treatment of breast, endometrial and prostate cancer and for the treatment of androgen-dependent skin diseases such as acne, alopecia and hirsutism. Various classes of STS-I chemotypes which have been developed are reviewed in Horvath et al., 2005, Expert Opin. Ther. Patents, 15(11),1541-1553*.*

Due to the severe threat for the embryo and the considerable costs associated with caring for infants born pre-term, it would be highly desirable to develop new active agents which prevent preterm birth. This need is further amplified by recent data suggesting an increase in preterm birth rates in most developed countries to 8 - 12% *(*Langhoff-Roos et a/., 2006, Bmj, 332(7547), 937-9*).*

### Summary of the Invention

The present invention is directed towards steroid sulfatase inhibitors useful in the treatment and/or prophylaxis of premature uterine contraction disorders such as pre-term labor. Notably, the invention is related to sulfamate steroid sulfatase inhibitors useful in the inhibition and/or reduction of premature uterine contractions.

A first aspect of the invention provides a use of a steroid sulfatase inhibitor or a pharmaceutically acceptable salt thereof or a pharmaceutically active derivative thereof, for the preparation of a pharmaceutical composition for the prevention and/or treatment of premature uterine contraction disorders.

A second aspect of the invention relates to a method for preventing and/or treating premature uterine contraction disorders in a patient. The method comprises administering a steroid sulfatase inhibitor, or a pharmaceutically acceptable salt or a pharmaceutically active derivative thereof in a patient in need thereof.

A third aspect of the invention relates to a steroid sulfatase inhibitor, as well as pharmaceutically acceptable salts thereof and pharmaceutically active derivatives thereof, for use in the prevention and/or treatment of premature uterine contraction disorders.

Other features and advantages of the invention will be apparent from the following detailed description.

### Detailed Description of the invention

The following paragraphs provide definitions of the various chemical moieties that make up the compounds according to the invention and are intended to apply uniformly throughout the specification and claims, unless an otherwise expressly set out definition provides a broader definition.

The term "alkyl" when used alone or in combination with other terms, comprises a straight chain or branched C₁-C₂₀ alkyl which refers to monovalent alkyl groups having 1 to 20 carbon atoms. This term is exemplified by groups such as methyl, ethyl, n-propyl, i-propyl, n-butyl, s-butyl, i-butyl, t-butyl, n-pentyl, 1-ethylpropyl, 2-methylbutyl, 3-methylbutyl, 2,2-dimethylpropyl, n-hexyl, 2-methylpentyl, 3-methylpentyl, 4-methylpentyl, n-heptyl, 2-methylhexyl, 3-methylhexyl, 4-niethylhexyl, 5-methylhexyl, n-heptyl, n-octyl, n-nonyl, n-decyl, tetrahydrogeranyl, n-dodecyl, n-tridecyl, n-tetradecyl, n-pentadecyl, n-hexadecyl, n-octadecyl, n-nonadecyl, and n-eicosanyl and the like. Preferably, these include C₁-C₉ alkyl, more preferably C₁-C₆ alkyl, especially preferably C₁-C₄ alkyl, which, by analogy, refer respectively to monovalent alkyl groups having I to 9 carbon atoms, monovalent alkyl groups having 1 to 6 carbon atoms and monovalent alkyl groups having 1 to 4 carbon atoms. Particularly, those include C₁-C₆ alkyl.

The term "alkenyl" when used alone or in combination with other terms, comprises a straight chain or branched C₂-C₂₀ alkenyl. Particularly, those include C₂-C₆ alkenyl which refers to groups having 2 to 6 carbon atoms and having at least 1 or 2 sites of alkenyl unsaturation. It may have any available number of double bonds in any available positions, and the configuration of the double bond may be the (E) or (Z) configuration. This term is exemplified by groups such as vinyl, allyl, isopropenyl, 1-propenyl, 2-methyl-1-propenyl, 1-butenyl, 2-butenyl, 3-butenyl, 2-ethyl-l-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, 1-heptenyl, 1-octenyl, geranyl, 1-decenyl, 1-tetradecenyl, 1-octadecenyl, 9-octadecenyl, 1-eicosenyl, and 3, 7, 11, 15-tetramethyl-1-hexadecenyl, and the like. Preferably, these include C₂-C₈ alkenyl, more preferably C₂-C₆ alkenyl. Among others, especially preferred are vinyl or ethenyl (-CH=CH₂), n-2-propenyl (ally, CH₂CH=CH₂), isopropenyl, 1-propenyl, 2-methyl-1-propenyl, 1-butenyl, 2-butenyl, and 3-methyl-2-butenyl and the like.

The term "alkynyl" when used alone or in combination with other terms, comprises a straight chain or branched C₂-C₂₀ alkynyl. It may have any available number of triple bonds in any available positions. This term is exemplified by groups such as alkynyl groups that may have a carbon number of 2-20, and optionally a double bond, such as ethynyl (-C≡CH), 1-propynyl, 2-propynyl (propargyl: -CH₂C≡CH), 2-butynyl, 2-pentene-4-ynyl, and the like. Particularly, these include C₂-C₈ alkynyl, more preferably C₂-C₆ alkynyl and the like. Preferably those include C₂-C₆ alkynyl which refers to groups having 2 to 6 carbon atoms and having at least 1 or 2 sites of alkynyl unsaturation.

The term "heteroalkyl" refers to C₁-C₁₂-alkyl, preferably C₁-C₆-alkyl, wherein at least one carbon has been replaced by a heteroatom selected from O, N or S, including 2-methoxy ethyl and the like.

The term "aryl" refers to an unsaturated aromatic carbocyclic group of from 6 to 14 carbon atoms having a single ring (*e*.*g*. phenyl) or multiple condensed rings (*e*.*g*., indenyl, naphthyl). Aryl include phenyl, naphthyl, anthryl, phenanthrenyl and the like.

The term "C₁-C₆ alkyl aryl" refers to aryl groups having a C₁-C₆ alkyl substituent, including methyl phenyl, ethyl phenyl and the like.

The term "aryl C₁-C₆ alkyl" refers to C₁-C₆ alkyl groups having an aryl substituent, including 3-phenylpropanyl, benzyl and the like.

The term "heteroaryl" refers to a monocyclic heteroaromatic, or a bicyclic or a tricyclic fused-ring heteroaromatic group. Particular examples of heteroaromatic groups include optionally substituted pyridyl, pyrrolyl, pyrimidinyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadia-zolyl, 1,2,5-oxadiazolyl, 1,3,4-oxadiazolyl,l,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxazolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl.

The term "C₁-C₆ alkyl heteroaryl" refers to heteroaryl groups having a C₁-C₆ alkyl substituent, including methyl furyl and the like.

The term "heteroaryl C₁-C₆ alkyl" refers to C₁-C₆ alkyl groups having a heteroaryl substituent, including furyl methyl and the like.

The term "C₂-C₆ alkenyl aryl" refers to an aryl groups having a C₂-C₆ alkenyl substituent, including vinyl phenyl and the like.

The term "aryl C₂-C₆ alkenyl" refers to a C₂-C₆ alkenyl groups having an aryl substituent, including phenyl vinyl and the like.

The term "C₂-C₆ alkenyl heteroaryl" refers to heteroaryl groups having a C₂-C₆ alkenyl substituent, including vinyl pyridinyl and the like.

The term "heteroaryl C₂-C₆ alkenyl" refers to C₂-C₆ alkenyl groups having a heteroaryl substituent, including pyridinyl vinyl and the like.

The term "C₃-C₈-cycloalkyl" refers to a saturated carbocyclic group of from 3 to 8 carbon atoms having a single ring (*e*.*g*. cyclohexyl) or multiple condensed rings (*e*.*g*. norbornyl). C₃-C₈-cycloalkyl includes cyclopentyl, cyclohexyl, norbornyl and the like.

The term "heterocycloalkyl" refers to a C₃-C₈-cycloalkyl group according to the definition above, in which up to 3 carbon atoms are replaced by heteroatoms chosen from the group consisting of O, S, NR, R being defined as hydrogen or methyl. Heterocycloalkyl include pyrrolidinyl, piperidinyl, piperazinyl, morpholinyl, tetrahydrofuranyl and the like.

The term "C₁-C₆ alkyl C₃-C₈-cycloalkyl" refers to C₃-C₈-cycloalkyl groups having a C₁-C₆ alkyl substituent, including methyl cyclopentyl and the like.

The term "C₃-C₈-cycloalkyl C₁-C₆ alkyl" refers to C₁-C₆ alkyl groups having a C₃-C₈-cycloalkyl substituent, including 3-cyclopentyl propyl and the like.

The term "C₁-C₆ alkyl heterocycloalkyl" refers to heterocycloalkyl groups having a C₁-C₆ alkyl substituent, including 4-methylpiperidinyl and the like.

The term "heterocycloalkyl C₁-C₆ alkyl" refers to C₁-C₆ alkyl groups having a heterocycloalkyl substituent, including (1-methylpiperidin-4-yl) methyl and the like.

The term "carboxy" refers to the group -C(O)OH.

The term "carboxy C₁-C₆ alkyl" refers to C₁-C₆ alkyl groups having a carboxy substituent, including 2-carboxyethyl and the like.

The term "acyl" refers to the group -C(O)R where R includes H, "alkyl," preferably "alkyl," "aryl," "heteroaryl," "C₃-C₈-cycloalkyl," "heterocycloalkyl," "aryl alkyl," "heteroaryl alkyl," "C₃-C₈-cycloalkyl alkyl" or "heterocycloalkyl alkyl", including acetyl and the like.

The term "acyl alkyl" to alkyl groups having an acyl substituent, including 2-acetylethyl and the like.

The term "acyl aryl" refers to aryl groups having an acyl substituent, including 2-acetylphenyl and the like.

The term "acyloxy" refers to the group -OC(O)R where R includes H, "alkyl", "alkenyl," "alkynyl," "C₃-C₈-cycloalkyl," "heterocycloalkyl," "aryl," "heteroaryl," "aryl alkyl", "heteroaryl alkyl," "aryl alkenyl," "heteroaryl alkenyl," "aryl alkynyl," "heteroaryl alkynyl," "C₃-C₈-cycloalkyl alkyl," or "heterocycloalkyl alkyl", including acetyloxy and the like.

The term "acyloxy alkyl" refers to alkyl groups having an acyloxy substituent, including 2-(ethylcarbonyloxy)ethyl and the like.

The term "alkoxy" refers to the group -O-R where R includes "alkyl", "aryl", "heteroaryl", "aryl alkyl" or "heteroaryl alkyl". Preferred alkoxy groups include for example, methoxy, ethoxy, phenoxy and the like.

The term "alkoxy alkyl" refers to alkyl groups having an alkoxy substituent, including methoxyethyl and the like.

The term "alkoxycarbonyl" refers to the group -C(O)OR where R includes "alkyl", "aryl", "heteroaryl" , "aryl alkyl", "heteroaryl alkyl" or "heteroalkyl".

The term "alkoxycarbonyl alkyl" refers to alkyl groups having an alkoxycarbonyl substituent, including 2-(benzyloxycarbonyl)ethyl and the like.

The term "aminocarbonyl" refers to the group -C(O)NRR' where R and R' are independently H, alkyl, aryl, heteroaryl, "aryl alkyl" or "heteroaryl alkyl," including N-phenyl carbonyl and the like.

The term "aminocarbonyl alkyl" refers to alkyl groups having an aminocarbonyl substituent, including 2-(dimethylaminocarbonyl)ethyl, N-ethyl acetamidyl, N,N-Diethylacetamidyl and the like.

The term "acylamino" refers to the group -NRC(O)R' where R and R' are independently H, "alkyl," "alkenyl," "alkynyl," "C₃-C₈-cycloalkyl," "heterocycloalkyl," "aryl," "heteroaryl," "aryl alkyl", "heteroaryl alkyl," "aryl alkenyl," "heteroaryl alkenyl," "aryl alkynyl," "heteroaryl alkynyl," "cycloalkyl alkyl," or "heterocycloalkyl alkyl", including acetylamino and the like.

The term "acylamino alkyl" refers to alkyl groups having an acylamino substituent, including 2-(propionylamino)ethyl and the like.

The term "ureido" refers to the group -NRC(O)NR'R" where R, R and R" are independently H, "alkyl," "alkenyl," "alkynyl," "C₃-C₈-cycloalkyl," "heterocycloalkyl," "aryl," "heteroaryl," "aryl alkyl", "heteroaryl alkyl," "aryl alkenyl," "heteroaryl alkenyl," "aryl alkynyl," "heteroaryl alkynyl," "cycloalkyl alkyl," or "heterocycloalkyl alkyl," and where R' and R," together with the nitrogen atom to which they are attached, can optionally form a 3-8-membered heterocycloalkyl ring.

The term "ureido alkyl" refers to -alkyl groups having an ureido substituent, including 2-(*N*'-methylureido)ethyl and the like.

The term "carbamate" refers to the group -NRC(O)OR' where R and R' are independently "alkyl," "alkenyl," "alkynyl," "C₃-C₈-cycloalkyl," "heterocycloalkyl," "aryl," "heteroaryl," "alkyl aryl" , "heteroaryl alkyl," "aryl alkenyl," "heteroaryl alkenyl," "aryl alkynyl," "heteroaryl alkynyl," "cycloalkyl alkyl," or "heterocycloalkyl alkyl" and optionally R can also be hydrogen.

The term "amino" refers to the group -NRR' where R and R' are independently H , "alkyl", "aryl", "heteroaryl", "alkyl aryl", "alkyl heteroaryl," "cycloalkyl," or "heterocycloalkyl," and where R and R', together with the nitrogen atom to which they are attached, can optionally form a 3-8-membered heterocycloalkyl ring.

The term "amino alkyl" refers to alkyl groups having an amino substituent, including 2-(1-pyrrolidinyl)ethyl and the like.

The term "ammonium" refers to a positively charged group -N⁺RR'R" where R, R' and R" are independently "alkyl", "alkyl aryl", "alkyl heteroaryl," "cycloalkyl," or "heterocycloalkyl," and where R and R', together with the nitrogen atom to which they are attached, can optionally form a 3-8-membered heterocycloalkyl ring.

The term "ammonium alkyl" refers to alkyl groups having an ammonium substituent, including 1-ethylpyrrolidinium and the like.

The term "halogen" refers to fluoro, chloro, bromo and iodo atoms.

The term "sulfonyloxy" refers to a group -OSO₂-R wherein R is selected from "alkyl," "alkyl" substituted with halogens, *e*.*g*., an -OSO₂-CF₃ group, "alkenyl," "alkynyl," "C₃-C₈-cycloalkyl," "heterocycloalkyl," "aryl," "heteroaryl," "aryl alkyl", "heteroaryl alkyl," "aryl alkenyl," "heteroaryl alkenyl," "aryl alkynyl," "heteroaryl alkynyl," "cycloalkyl alkyl," or "heterocycloalkyl alkyl".

The term "sulfamate" refers to a group -OSO₂-NRR' wherein R and R' are independently selected from H, "alkyl," "alkenyl," "alkynyl," "C₃-C₈-cycloalkyl," "heterocycloalkyl," "aryl," "heteroaryl," "aryl alkyl", "heteroaryl alkyl," "aryl alkenyl," "heteroaryl alkenyl," "aryl alkynyl," "heteroaryl alkynyl," "C₃-C₈-cycloalkyl alkyl," or "heterocycloalkyl alkyl" and the like.

The term "sulfonyloxy alkyl" refers to alkyl groups having a sulfonyloxy substituent, including 2-(methylsulfonyloxy)ethyl and the like.

The term "sulfonyl" refers to group "-SO₂-R" wherein R is selected from "aryl," "heteroaryl," "alkyl," "alkyl" substituted with halogens, *e*.*g*., an -SO₂-CF₃ group, "alkenyl," "alkynyl," "C₃-C₈-cycloalkyl," "heterocycloalkyl," "aryl," "heteroaryl," "aryl alkyl", "heteroaryl alkyl," "aryl alkenyl," "heteroaryl alkenyl," "aryl alkynyl," "heteroaryl alkynyl," "cycloalkyl alkyl," or "heterocycloalkyl alkyl".

The term "sulfonyl alkyl" refers to alkyl groups having a sulfonyl substituent, including 2-(methylsulfonyl)ethyl and the like.

The term "sulfinyl" refers to a group "-S(O)-R" wherein R is selected from "alkyl," "alkyl" substituted with halogens, *e*.*g*., a-SO-CF₃ group, "alkenyl," "alkynyl," "C₃-C₈-cycloalkyl," "heterocycloalkyl," "aryl," "heteroaryl," "aryl alkyl", "heteroaryl alkyl," "aryl alkenyl," "heteroaryl alkenyl," "aryl alkynyl," "heteroaryl alkynyl," "C₃-C₈-cycloalkyl alkyl," or "heterocycloalkyl alkyl".

The term "sulfinyl alkyl" refers to alkyl groups having a sulfinyl substituent, including 2-(methylsulfinyl)ethyl and the like.

The term "sulfanyl" refers to groups -S-R where R includes H, "alkyl," "alkyl" substituted with halogens, *e*.*g*., a -S-CF₃ group, "alkenyl," "alkynyl," "C₃-C₈-cycloalkyl," "heterocycloalkyl," "aryl," "heteroaryl," "aryl alkyl", "heteroaryl alkyl," "aryl alkenyl," "heteroaryl alkenyl," "aryl alkynyl," "alkynylheteroaryl," "cycloalkyl alkyl," or "heterocycloalkyl alkyl". Preferred sulfanyl groups include methylsulfanyl, ethylsulfanyl, and the like.

The term "sulfanyl alkyl" refers to C₁-C₅-alkyl groups having a sulfanyl substituent, including 2-(ethylsulfanyl)ethyl and the like.

The term "sulfonylamino" refers to a group -NRSO₂-R' where R and R' are independently "alkyl," "alkenyl," "alkynyl," "C₃-C₈-cycloalkyl," "heterocycloalkyl," "aryl," "heteroaryl," "aryl alkyl", "heteroaryl alkyl," "aryl alkenyl," "heteroaryl alkenyl," "aryl alkynyl," "heteroaryl alkynyl," "C₃-C₈-cycloalkyl alkyl," or "heterocycloalkyl alkyl".

The term "sulfonylamino alkyl" refers to alkyl groups having a sulfonylamino substituent, including 2-(ethylsulfonylamino)ethyl and the like.

The term "aminosulfonyl" refers to a group -SO₂-NRR' where R and R' are independently H, "alkyl," "alkenyl," "alkynyl," "C₃-C₈-cycloalkyl," "heterocycloalkyl," "aryl," "heteroaryl," "aryl alkyl", "heteroaryl alkyl," "aryl alkenyl," "heteroaryl alkenyl," "aryl alkynyl," "heteroaryl alkynyl," "C₃-C₈-cycloalkyl alkyl," or "heterocycloalkyl alkyl", and where R and R', together with the nitrogen atom to which they are attached, can optionally form a 3-8-membered heterocycloalkyl ring. Aminosulfonyl groups include cyclohexylaminosulfonyl, piperidinylsulfonyl and the like.

The term "aminosulfonyl alkyl" refers to alkyl groups having an aminosulfonyl substituent, including 2-(cyclohexylaminosulfonyl)ethyl and the like.

Unless otherwise constrained by the definition of the individual substituent, the term "substituted" refers to groups substituted with from 1 to 5 substituents selected from the group consisting of "alkyl," "alkenyl," "alkynyl," "C₃-C₈-cycloalkyl," "heterocycloalkyl," "alkyl aryl," "alkyl heteroaryl," "alkyl cycloalkyl," "alkyl heterocycloalkyl," "amino," "aminosulfonyl," "ammonium," "acyl amino," "amino carbonyl," "aryl," "heteroaryl," "sulfinyl," "sulfonyl," "alkoxy," "alkoxy carbonyl," "carbamate," "sulfanyl," "halogen," trihalomethyl, cyano, hydroxy, mercapto, nitro, and the like.

The term "pharmaceutically acceptable salts or complexes" refers to salts or complexes of the below-specified steroid sulfatase inhibitors. Examples of such salts include, but are not restricted, to base addition salts formed by reaction of steroid sulfatase inhibitors with organic or inorganic bases such as hydroxide, carbonate or bicarbonate of a metal cation such as those selected in the group consisting of alkali metals (sodium, potassium or lithium), alkaline earth metals (e.g. calcium or magnesium), or with an organic primary, secondary or tertiary alkyl amine. Amine salts derived from methylamine, dimethylamine, trimethylamine, ethylamine, diethylamine, triethylamine, morpholine, N-Me-D-glucamine, N,N'-bis(phenylmethyl)-1,2-ethanediamine, tromethamine, ethanolamine, diethanolamine, ethylenediamine, N-tnethylmorpholine, procaine, piperidine, piperazine and the like are contemplated being within the scope of the instant invention.

Also comprised are salts which are formed from to acid addition salts formed with inorganic acids (e.g. hydrochloric acid, hydrobromic acid, sulfuric acid, phosphoric acid, nitric acid, and the like), as well as salts formed with organic acids such as acetic acid, oxalic acid, tartaric acid, succinic acid, malic acid, fumaric acid, maleic acid, ascorbic acid, benzoic acid, tannic acid, palmoic acid, alginic acid, polyglutamic acid, naphthalene sulfonic acid, naphthalene disulfonic acid, and poly-galacturonic acid.
"Pharmaceutically active derivative" refers to any compound that upon administration to the recipient, is capable of providing directly or indirectly, the activity disclosed herein. The term "indirectly" also encompasses prodrugs which may be converted to the active form of the drug via endogenous enzymes or metabolism. The prodrug is a derivative of the compounds according to the invention and presenting premature uterine contraction inhibitory activity that has a chemically or metabolically decomposable group, and a compound that may be converted into a pharmaceutically active compound *in vivo* by solvolysis under physiological conditions.

The term "premature uterine contraction" includes "pre-term labour" which means a disease or condition where the onset of labour occurs after the gestation viability and before 37 completed weeks of pregnancy. It is characterized by the presence of uterine contractions of sufficient frequency and intensity to effect progressive effacement and dilation of the cervix prior to term gestation (typically between 20 and 37 week). Several factors may lead to pre-term labor or constitute pre-term labor condition predispositions such as for example preeclampsia (also known as toxemia or high blood pressure of pregnancy), infections in the cervix or uterus (such as group B streptococcus, urinary tract infections, vaginal infections, infections of the foetal/placental tissues), cervical incompetence (inability of the cervix to stay closed during pregnancy), multiple gestation (twins, triplets etc...), mother's age (over the age of 35).

The term "steroid sulfatase inhibitor" (STS-I) means a compound capable of inhibiting a steroid sulfatase enzyme (E.C.3.1.6.2). In particular, a sulfatase inhibitor (STS-I) is defined as being a compound that prevents active estrogens to be formed from their biologically inactive sulfated forms, and active androgens to be formed from their biologically inactive sulfated forms by inhibiting the steroid sulfatase enzyme. Typically, when incubated with a steroid sulfatase enzyme (E.C.3.1.6.2) at a pH 7.4 and 37°C, a sulfatase inhibitor according to the invention would provide an affinity constant (Km) value of less than 50 mM. STS inhibitory activity can be assayed, using 3H oestrone sulphate as described in WO 96/15257, for example. Further, the STS inhibitory activity can be assayed through the ability to inhibit oestrone sulfatase activity using either intact JEG3 choriocarcinoma cells or placental microsomes such as described in Purohit et al., 1999, J Steroid Biochem. Mol. Biol. 69 (1-6):227-238*.* It is to be noted that other assays could be used to determine STS activity and thus STS inhibition. For example, reference may also be made to the teachings of WO 99/50453.

The term "inhibitor" used in the context of the invention is defined as a molecule that inhibits completely or partially the pre-term uterine contractions (by decreasing the frequency and/or amplitude of the contractions, for example).

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or relieving the disease, i.e., causing regression of the disease and/or its symptoms or conditions.

The term "subject" as used herein refers to mammals. For examples, mammals contemplated by the present invention include human, primates, domesticated animals such as cattle, sheep, pigs, horses and the like.

### Compounds

The steroid sulfatase inhibitor used in the manufacture of a medicament for the prevention or treatment of premature uterine contractions, is capable of inhibiting a steroid sulfatase enzyme (E.C.3.1.6.2). The steroid sulfatase inhibitor according to the invention may be any suitable compound. Examples of steroid sulfatase inhibitors are presented in *Horvath et al., 2005, above;* Nussbaumer et al., 2004, Medicinal Research Reviews 24(4), 529-576*:* Poirier et al., 1999, Expert Opin. Ther. Patents, 9(8), 1083-1099*;* Nussbaumer et al., 2003, Expert Opin. Ther. Patents, 13(5), 605-625 *and* Reed et al., 2005, Endocrine Rev., 26(2), 171-202*.*

In one embodiment, steroid sulfatase inhibitor according to the invention comprises a sulfamate group. In this aspect, the steroid sulfatase inhibitor is referred to as a sulfamate compound. The term "sulfamate" includes an ester of sulphamic acid, or an ester of an N-substituted derivative of sulphamic acid, or a salt thereof. The sulfamate group preferably has the Formula (I) as described below.

### Compositions

The invention provides steroid sulfatase inhibitor pharmaceutical compositions useful for the treatment of premature uterine contractions. The invention further provides methods for treating a mammalian patient, and most preferably a human patient, who is suffering from premature uterine contractions.

Pharmaceutical compositions of the invention can contain one or more steroid sulfatase inhibitor(s) in any form described herein. Compositions of this invention may further comprise one or more pharmaceutically acceptable additional ingredient(s), such as alum, stabilizers, antimicrobial agents, buffers, coloring agents, flavoring agents, adjuvants, and the like.

The compounds of the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed. Compositions according to the invention are preferably injectable.

Compositions of this invention may also be liquid formulations, including, but not limited to, aqueous or oily suspensions, solutions, emulsions, syrups, and elixirs. Liquid forms suitable for oral administration may include a suitable aqueous or non-aqueous vehicle with buffers, suspending and dispensing agents, colorants, flavors and the like. The compositions may also be formulated as a dry product for reconstitution with water or other suitable vehicle before use. Such liquid preparations may contain additives, including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Non-aqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in Part 5 *of* Remington's Pharmaceutical Sciences, 21st Edition, 2005, University of the Sciences in Philadelphia, Lippincott Williams & Wilkins, which is incorporated herein by reference.

Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

Injectable compositions are typically based upon injectable sterile saline or phosphate-buffered saline or other injectable carriers known in the art.

Compositions of this invention may also be formulated as suppositories, which may contain suppository bases including, but not limited to, cocoa butter or glycerides. Compositions of this invention may also be formulated for inhalation, which may be in a form including, but not limited to, a solution, suspension, or emulsion that may be administered as a dry powder or in the form of an aerosol using a propellant, such as dichlorodifluoromethane or trichlorofluoromethane. Compositions of this invention may also be formulated transdermal formulations comprising aqueous or non-aqueous vehicles including, but not limited to, creams, ointments, lotions, pastes, medicated plaster, patch, or membrane.

Compositions of this invention may also be formulated for parenteral administration, including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

Compositions of this invention may also be formulated as a liposome preparation. The liposome preparation can comprise liposomes which penetrate the cells of interest or the *stratum corneum*, and fuse with the cell membrane, resulting in delivery of the contents of the liposome into the cell. Other suitable formulations can employ niosomes. Niosomes are lipid vesicles similar to liposomes, with membranes consisting largely of non-ionic lipids, some forms of which are effective for transporting compounds across the *stratum corneum.*

The compounds of this invention can also be administered in sustained release forms or from sustained release drug delivery systems. A description of representative sustained release materials can also be found in the incorporated materials in *Remington's Pharmaceutical Sciences.*

### Mode of administration

Compositions of this invention may be administered in any manner, including, but not limited to, orally, parenterally, sublingually, transdermally, vaginally, rectally, transmucosally, topically, via inhalation, via buccal or intranasal administration, or combinations thereof. Parenteral administration includes, but is not limited to, intravenous, intra-arterial, intra-peritoneal, subcutaneous, intramuscular, intra-thecal, and intra-articular. The compositions of this invention may also be administered in the form of an implant, which allows slow release of the compositions as well as a slow controlled i.v. infusion. In a preferred embodiment, steroid sulfatase inhibitors according to the invention are administered orally.

This invention is further illustrated by the following examples that are not intended to limit the scope of the invention in any way.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

### Combination

According to the invention, the steroid sulfatase inhibitor and pharmaceutical formulations thereof can be administered alone or in combination with a co-agent useful in the treatment of premature uterine contractions, such as substances useful in the treatment and/or prevention of pre-term labor e.g. for example a co-agent selected from tocolytic agents such as Oxytocin receptor inhibitors (e.g. atosiban) and Progesterone or Progestins, Beta-2 agonists (e.g. ritodrine), Cyclo-oxygenase inhibitors (e.g. indomethacin), Calcium channel blockers (e.g. nifedipine) or antibiotics.

The invention encompasses the administration of a steroid sulfatase inhibitor or of a pharmaceutical formulation thereof, wherein the steroid sulfatase inhibitor or the pharmaceutical formulation thereof is administered to an individual prior to, simultaneously or sequentially with other therapeutic regimens or co-agents useful in the treatment of premature uterine contractions (e.g. multiple drug regimens), in a therapeutically effective amount. Steroid sulfatase inhibitors or the pharmaceutical formulations thereof that are administered simultaneously with said co-agents can be administered in the same or different composition(s) and by the same or different route(s) of administration.

### Patients

In an embodiment, patients according to the invention are patients suffering from premature uterine contractions.

In a further embodiment, patients according to the invention are patients suffering from preterm labor.

In another embodiment, patients according to the invention are patients with a high risk of presenting pre-term labor such as patient suffering from multiple pregnancy, history of preterm labor, premature rupture of membrane, hypertensive disorders of pregnancy, intrauterine growth restriction, antepartum haemorrhage, hydramnios, cervical incompetence and uterine malformation.

### Use according to the invention

In one embodiment, the invention provides a use of a steroid sulfatase inhibitor, as well as pharmaceutically acceptable salts and pharmaceutically active derivative thereof, for the preparation of a pharmaceutical composition for the treatment or prophylaxis of a disease or condition associated with premature uterine contractions.

In a further embodiment, the invention provides a use according to the invention, wherein the steroid sulfatase inhibitor is a sulfamate.

In another further embodiment, the invention provides a use according to the invention, wherein the steroid sulfatase inhibitor is a sulfamate of Formula (I): 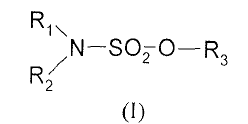 wherein R¹ and R² are independently selected from H, optionally substituted C₁-C₆ alkyl, optionally substituted C₂-C₆ alkenyl, optionally substituted C₂-C₆ alkynyl, optionally substituted alkoxy, optionally substituted C₃-C₈-cycloalkyl, optionally substituted heterocycloalkyl, optionally substituted acyl, optionally substituted aryl, optionally substituted heteroaryl, optionally substituted C₁-C₆ alkyl aryl, optionally substituted C₁-C₆ alkyl heteroaryl, optionally substituted C₁-C₆ alkyl C₃-C₈-cycloalkyl, optionally substituted C₁-C₆ alkyl heterocycloalkyl, optionally substituted aryl C₁-C₆ alkyl, optionally substituted heteroaryl C₁-C₆ alkyl, optionally substituted C₃-C₈-cycloalkyl C₁-C₆ alkyl and optionally substituted heterocycloalkyl C₁-C₆ alkyl or R¹ and R² form together an optionally substituted C₂-C₆ alkenyl group; R³ is a group selected from a group of Formulae (II), (III) and (IV): 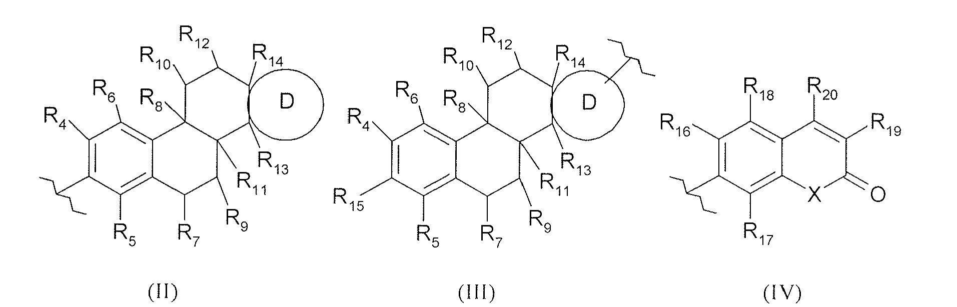 wherein R⁴ and R⁵ are independently selected from H; halogen; nitro; optionally substituted amino; optionally substituted sulfanyl (e.g. SCH₃ or SC₂H₅); optionally substituted acyl; optionally substituted alkoxy such as optionally substituted methoxy; optionally substituted thioalkyl; optionally substituted C₁-C₆ alkyl, such as optionally substituted ethyl (e.g. C₂H₅), optionally substituted methyl such as optionally substituted halogeno methyl (e.g. CF₂H); optionally substituted C₂-C₆ alkenyl; optionally substituted alkoxy alkyl such as optionally substituted methoxymethyl; optionally substituted amino alkyl; optionally substituted C₃-C₈-cycloalkyl; and optionally substituted aryl; R⁶ R⁷, R⁸, R⁹, R¹⁰, R¹¹, R¹², R¹³ and R¹⁴ are independently selected from H; OH; halogen and optionally substituted C₁-C₆ alkyl such as optionally substituted methyl; R¹⁵ is selected from selected from H; OH; halogen; sulfamate and optionally substituted C₁-C₆ alkyl such as optionally substituted methyl; R¹⁶, R¹⁷ and R¹⁸ are independently selected from H; OH; halogen; optionally substituted C₁-C₆ alkyl; optionally substituted C₂-C₆ alkenyl; optionally substituted alkoxy; optionally substituted acylamino; optionally substituted amino and optionally substituted sulfonyl; R¹⁹ and R²⁰ are independently selected from H; OH; halogen; optionally substituted C₁-C₆ alkyl such as optionally substituted methyl (e.g. 4-methyl, 3,4 dimethyl) and optionally substituted propyl; optionally substituted C₂-C₆ alkenyl; optionally substituted alkoxy; optionally substituted acylamino; optionally substituted amino and optionally substituted sulfonyl; or R¹⁹ and R²⁰ form together an optionally substituted C₃-C₁₄-cydoalkyl ring; R²¹ is selected from H and optionally substituted C₁-C₆ alkyl; R²² and R²³ are independently selected from H; OH; halogen; optionally substituted C₁-C₆ alkyl; D is an optionally substituted ring selected from optionally substituted C₃-C₈-cycloalkyl and optionally substituted heterocycloalkyl; X is selected from O, NR²¹ and CR²²R²³.

In a particular embodiment, the invention provides a use according to the invention wherein the steroid sulfatase inhibitor is a sulfamate of Formula (I) wherein R¹ and R² are independently selected from H and optionally substituted C₁-C₆ alkyl.

In a particular embodiment, the invention provides a use according to the invention wherein the steroid sulfatase inhibitor is a sulfamate of Formula (I) wherein at least one of R¹ and R² is H.

In a further particular embodiment, the invention provides a use according to the invention wherein the steroid sulfatase inhibitor is a sulfamate of Formula (I) wherein each of R¹ and R² is H.

In another particular embodiment, the invention provides a use according to the invention wherein the steroid sulfatase inhibitor is a sulfamate of Formula (I) wherein R³ is of Formula (II).

In a further particular embodiment, the invention provides a use according to the invention wherein the steroid sulfatase inhibitor is a sulfamate of Formula (I) wherein R³ is of Formula (II'): 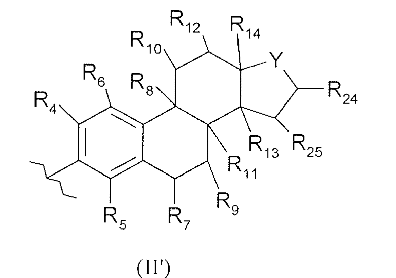 wherein R⁴ , R⁵, R⁶, R⁷, R⁸, R⁹, R¹⁰, R¹¹, R¹², R¹³ and R¹⁴ are as defined above; R²⁴ and R²⁵ are independently selected from H; OH; halogen; optionally substituted C₁-C₆ alkyl; optionally substituted C₂-C₆ alkenyl; optionally substituted alkoxy; optionally substituted acylamino; optionally substituted amino and optionally substituted sulfonyl; Y is selected from optionally substituted acyl; optionally substituted alkoxy; -C(O)-; optionally substituted C₂-C₆ alkyl; optionally substituted C₂-C₆ alkenyl; optionally substituted C₂-C₆ alkynyl; optionally substituted C=N-OR²⁹ and CR²⁶R²⁷; R²⁶ and R²⁷ are independently selected from H; OH; nitrile; optionally substituted sulfamate; optionally substituted alkoxy; optionally substituted carbonyl; optionally substituted amino; optionally substituted nitrile; optionally substituted C₁-C₆ alkyl; optionally substituted C₂-C₆ alkenyl; optionally substituted aryl; optionally substituted heteroaryl; optionally substituted aryl C₁-C₆ alkyl such as optionally substituted phenyl C₁-C₆ alkyl (e.g. alkyl phenyl methyl like 4-ter-butyl phenyl methyl, halogenophenyl methyl like 3-bromophenyl methyl; phenyl methyl); optionally substituted aminocarbonyl; optionally substituted acylamino; optionally substituted alkoxycarbonyl; and optionally substituted sulfonyloxy; R²⁹ is selected from H; optionally substituted C₁-C₆ alkyl and optionally substituted carbonyl.

In another further particular embodiment, the invention provides a use according to the invention wherein the steroid sulfatase inhibitor is a sulfamate of Formula (I) wherein R³ is of Formula (II"): 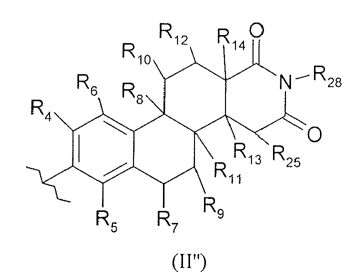 wherein R⁴ R⁵, R⁶, R⁷, R⁸, R⁹, R¹⁰, R¹¹, R¹², R¹³, R¹⁴ and R²⁵ are as defined above; R²⁸ is selected from H; OH; optionally substituted C₁-C₆ alkyl such as optionally substituted methyl, optionally substituted ethyl, optionally substituted propyl, optionally substituted pentyl (e.g. n-pentyl, bromo pentyl), optionally substituted hexyl; optionally substituted C₂-C₆ alkenyl such as propylenyl; optionally substituted aryl C₁-C₆ alkyl such as optionally substituted benzyl (benzyl, 4-t-butyl benzyl); optionally substituted heteroaryl C₁-C₆ alkyl such as optionally substituted pyridine methyl (e.g. (3-pyridinyl)methyl); optionally substituted C₃-C₈-cycloalkyl C₁-C₆ alkyl such as optionally substituted cyclopropyl methyl; optionally substituted aryl; optionally substituted heteroaryl; optionally substituted C₃-C₈-cycloalkyl; and optionally substituted heterocycloalkyl.

In another further particular embodiment, the invention provides a use according to the invention wherein the steroid sulfatase inhibitor is a sulfamate of Formula (I) wherein R³ is of Formula (II'''): 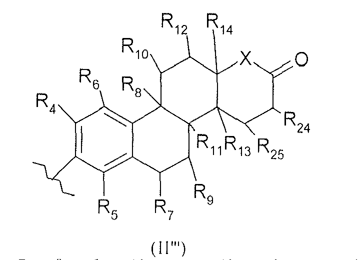 wherein R⁴, R⁵, R⁶, R⁷, R⁸, R⁹, R¹⁰, R¹¹, R¹², R¹³, R¹⁴, R²⁴ and R²⁵ are as defined above; X is selected from N and O.

In another particular embodiment, the invention provides a use according to the invention wherein the steroid sulfatase inhibitor is a sulfamate of Formula (I) wherein R³ is of Formula (III).

In a further particular embodiment, the invention provides a use according to the invention wherein the steroid sulfatase inhibitor is a sulfamate of Formula (I) wherein R³ is of Formula (III'): 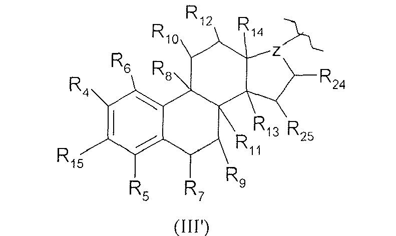 wherein R⁴ R⁵, R⁶, R⁷, R⁸, R⁹, R¹⁰, R¹¹, R¹², R¹³, R¹⁴, R¹⁵, R²⁴ and R²⁵ are as defined above; Z is CR³⁰; R³⁰ is selected from H and optionally substituted C₁-C₆ alkyl.

In another particular embodiment, the invention provides a use according to the invention wherein the steroid sulfatase inhibitor is a sulfamate of Formula (I) wherein R³ is of Formula (IV).

In a further particular embodiment, the invention provides a use according to the invention wherein the steroid sulfatase inhibitor is a sulfamate of Formula (I) wherein R³ is of Formula (IV'): 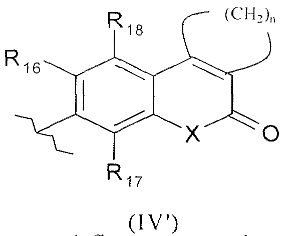 wherein R¹⁶, R¹⁷ and R¹⁸ are as defined above; n is an integer selected from 3 to 14, notably from 3 to 10, such as from 3 to 5.

In a further particular embodiment, the invention provides a use according to the invention wherein the steroid sulfatase inhibitor is a sulfamate of Formula (I) wherein R³ is of Formula (IV') and wherein wherein R¹⁶, R¹⁷ and R¹⁸ are as defined above; n is 5.

In another particular embodiment, the invention provides a use according to the invention wherein the steroid sulfatase inhibitor is a sulfamate of Formula (I) wherein R⁶ R⁷, R⁸, R⁹, R¹⁰, R¹¹, R¹² and R¹³ are H.

In another particular embodiment, the invention provides a use according to the invention wherein the steroid sulfatase inhibitor is a sulfamate of Formula (I) wherein R³ is of Formula (II) or (III) and R¹⁴ is optionally substituted C₁-C₆ alkyl.

In a further particular embodiment, the invention provides a use according to the invention wherein the steroid sulfatase inhibitor is a sulfamate of Formula (I) wherein R³ is of Formula (II) or (III) and wherein R¹⁴ is methyl.

In another particular embodiment, the invention provides a use according to the invention wherein the steroid sulfatase inhibitor is a sulfamate of Formula (I) wherein R³ is of Formula (II') and Y is selected from -C(O)-, C(OH)R²⁷ and CHR²⁷.

In another particular embodiment, the invention provides a use according to the invention wherein the steroid sulfatase inhibitor is a sulfamate of Formula (I) wherein R³ is of Formula (II") and R²⁸ is selected from optionally substituted C₁-C₆ alkyl and optionally substituted C₂-C₆ alkenyl.

In another particular embodiment, the invention provides a use according to the invention wherein the steroid sulfatase inhibitor is a sulfamate of Formula (I) wherein R³ is of Formula (II") and R²⁸ is H.

In another particular embodiment, the invention provides a use according to the invention wherein the steroid sulfatase inhibitor is a sulfamate of Formula (I) wherein R³ is of Formula (III') and R¹⁵ is optionally substituted alkoxy.

In another particular embodiment, the invention provides a use according to the invention wherein the steroid sulfatase inhibitor is a sulfamate of Formula (I) wherein R³ is of Formula (III') and R¹⁵ is methoxy.

In another particular embodiment, the invention provides a use according to the invention wherein the steroid sulfatase inhibitor is a sulfamate of Formula (I) wherein R³ is of Formula (IV); R¹⁶, R¹⁷ and R¹⁸ are H.

In another particular embodiment, the invention provides a use according to the invention wherein the steroid sulfatase inhibitor is a sulfamate of Formula (I) wherein R³ is of Formula (IV') and n is selected from 3 to 7.

In a further particular embodiment, the invention provides a use according to the invention wherein the steroid sulfatase inhibitor is a sulfamate of Formula (I) wherein R³ is of Formula (IV') wherein n is an integer selected from 3 to 5.

In a particular embodiment, steroid sulfatase inhibitors of the invention include in particular those of Formula (I) wherein R³ is of Formula (II') and selected from the following group: 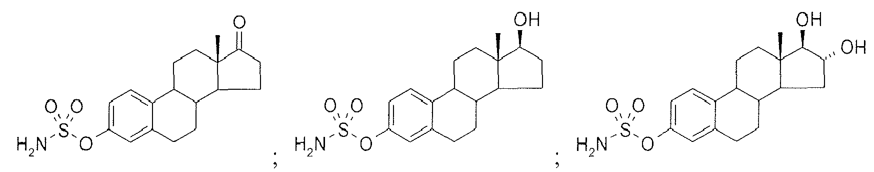 and

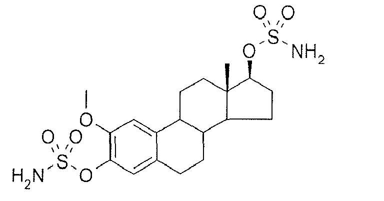

In another particular embodiment, steroid sulfatase inhibitors of the invention include in particular those of Formula (I) wherein R³ is of Formula (II") and selected from the following group: 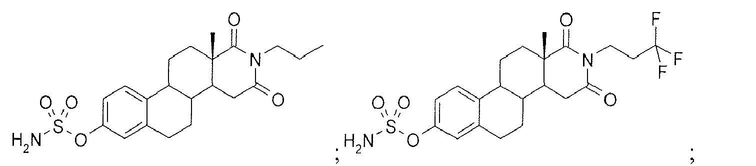 and

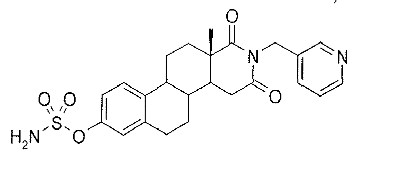

In another particular embodiment, steroid sulfatase inhibitors of the invention include in particular those of Formula (I) wherein R³ is of Formula (III') such as the following compound:

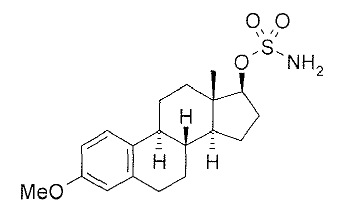

In another particular embodiment, steroid sulfatase inhibitors of the invention include in particular those of Formula (I) wherein R³ is of Formula (IV') and selected from the following group:

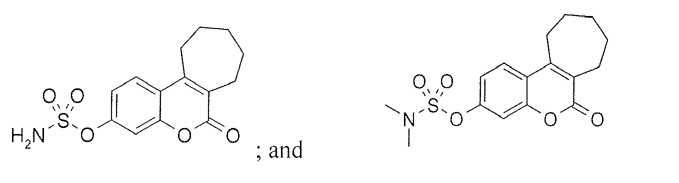

In another embodiment, the invention provides a method for preventing or treating premature uterine contractions in a patient. The method comprises administering a steroid sulfatase inhibitor, or a pharmaceutically acceptable salt or a pharmaceutically active derivative thereof in a patient in need thereof.

In a further embodiment, the invention provides a method according to the invention wherein the steroid sulfatase inhibitor is a sulfamate.

In a further embodiment, the invention provides a method according to the invention wherein the steroid sulfatase inhibitor is a sulfamate according to Formula (I).

In another embodiment, the invention provides a steroid sulfatase inhibitor, as well as pharmaceutically acceptable salts and pharmaceutically active derivatives thereof, for use in the treatment or prophylaxis of premature uterine contractions.

In another embodiment, the invention provides a use, a steroid sulfatase inhibitor or a method according to the invention wherein the premature uterine contraction condition is pre-term labor.

Steroid sulfatase inhibitors according to the present invention also comprise their tautomers, their geometrical isomers, their optically active forms as enantiomers, diastereomers and their racemate forms, as well as pharmaceutically acceptable salts thereof.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.

### Synthesis of steroid sulfatase inhibitors:

The steroid sulfatase inhibitors can be prepared from readily available starting materials using methods and procedures known from the skilled person. It will be appreciated that where typical or preferred experimental conditions (i.e. reaction temperatures, time, moles of reagents, solvents etc.) are given, other experimental conditions can also be used unless otherwise stated. Optimum reaction conditions may vary with the particular reactants or solvents used, but such conditions can be determined by the person skilled in the art, using routine optimisation procedures. Synthetic approaches for obtaining compounds of steroid sulfatase inhibitors and notably sulfamates are described in WO 2007/099304*;* WO 04/085459*;* WO 03/033518*;* WO 96/05216*;* Woo et al., 2000, Chemistry & Biology, 7(10), 773-791*;* Fischer et a/., 2003, Biorg Med Chem, 1685; Leese et al., 2005, J. Med. Chem., 48, 5243-5256 *and* Horwarth et al., 1994, J. Med. Chem., 37, 219-221*.*

If the above synthetic methods are not applicable to obtain steroid sulfatase inhibitors according to the invention and/or necessary intermediates, suitable methods of preparation known by a person skilled in the art should be used. In general, the synthesis pathways for any individual steroid sulfatase inhibitor will depend on the specific substituents of each molecule and upon the ready availability of intermediates necessary; again such factors being appreciated by those of ordinary skill in the art. For all the protection and deprotection methods, see Philip J. Kocienski, in "Protecting Groups", Georg Thieme Verlag Stuttgart, 2005 and Theodora W. Greene and Peter G. M. Wuts in "Protective Groups in Organic Synthesis", Wiley Interscience, 4th Edition 2006*.* Compounds of this invention can be isolated in association with solvent molecules by crystallization from evaporation of an appropriate solvent. The pharmaceutically acceptable acid addition salts of the steroid sulfatase inhibitors, which contain a basic center, may be prepared in a conventional manner. For example, a solution of the free base may be treated with a suitable acid, either neat or in a suitable solution, and the resulting salt isolated either by filtration or by evaporation under vacuum of the reaction solvent. Pharmaceutically acceptable base addition salts may be obtained in an analogous manner by treating a solution of a steroid sulfatase inhibitor with a suitable base. Both types of salts may be formed or interconverted using ion-exchange resin techniques.

In the following the present invention shall be illustrated by means of some examples, which are not to be viewed as limiting the scope of the invention.

### The following abbreviations refer respectively to the definitions below:

**dGA** (day of gestational age), **h** (hour), **IM** (intramuscular), **i.g.** (intragastric), **i.v.** (intravenous), **kg** (kilogram), **MBq** (megabecquerel), **µm** (micrometer), **mg** (milligram), **min** (minute), **mU** (milliunit), **p.c.** (post-coïtum), **ACTH,** (Adreno CorticoTropic Hormone), **AF** (amniotic fluid), **CRF** (Cortico-Releasing Factor), **DHEAS** (Dehydroepiandrosterone Sulphate), **EDTA** (Ethylenediaminetetraacetic acid), **EIS** (Oestrone sulphate), **IP** (Imaging Plate), **LC** (Liquid chromatography), **MS** (Mass spectrometry), **OT** (Oxytocin), **PBMC** (Peripheral Blood Mononuclear Cell), **STS** (Steroid sulfatase), **STS-I** (Steroid sulfatase inhibitor).

### Example 1: Placental barrier crossing ability measurement

The ability of a steroid sulfatase inhibitor according to the invention to cross the placental barrier can be investigated as follows. This assay was performed with a steroid sulfatase inhibitor according to the invention (Estradiol Sulfamate or E2MATE) which is used for the treatment of symptoms related to estrogen deficiency in postmenopausal or hysterectomized women.

Six female albino rats received a single intravenous bolus dose of 0.47 mg ¹⁴C-labeled Estradiol Sulfamate per kg. Six female albino, 3 pregnant albino and 4 female pigmented rats received 1 mg ¹⁴C-labeled Estradiol Sulfamate per kg as an intragastic dose. Animals were sacrificed at different time points (female albino i.v.: 15 min, 1 h, 3 h, 7 h, 1 day and 7 day; female albino i.g.: 15 min, 1 h, 3 h, 7 h, 1 day and 7 day; pregnant (18 day p.c.) albino i.g.: 1 h, 7 h and 1 day and female pigmented i.g.: 1 h, 1 day, 7 day and 14 day - the 14 day animal was exempt from sectioning, because no hint of binding to melanin containing structures was observed in the 7 day animal). Animals were deeply frozen in a mixture of hexane and dry ice and embedded into a block of carboxymethyl cellulose, which was frozen and stored at approximately -20°C. External standards were used for semiquantitative evaluation of radioluminograms and were prepared by spiking an erythrocyte concentrate with different concentrations of ¹⁴C-radioactivity. Pores were drilled into a separate block of carboxymethyl cellulose, filled with external standards and frozen at approximately -20°C. Whole body sagittal sections of 50 µm thickness were taken through each animal and the external standard block using a Leica CM 3600 cryomicrotome. After freeze drying of the sections in the cryostat for a minimum time of 48 hours, they were exposed to an Imaging Plate (IP) for a period of 4 to 24 hours and the IP was scanned using the bioimaging analyzer BAS 2000. For evaluation, the external standard samples were related to a colored scale using the software AIDA (Advanced Image Data Analysis, version 3.10, Raytest, Germany), each color representing a specific radioactive concentration (MBq). The different tissues were compared semi-quantitatively to the external standards and concentration ranges were determined.

Distribution of radiolabel in pregnant albino animals was similar compared to the distribution in female albino rats following intra-gastric administration. At 1 h post-dose ¹⁴C-concentrations were slightly higher in pregnant animals and after 7 h the general radiolabel concentration was lower than in non-pregnant animals, ¹⁴C-radiolabel concentrations in the foetus, foetal liver and placenta were in the high range at 1 h post-dose. Mammary gland and foetal membranes showed medium concentrations. At 7 h and 1 day post dose ¹⁴C-concentrations decreased, but were still between the higher low and the lower high range concentrations.

This indicated that a steroid sulfatase inhibitor according to the invention (Estradiol Sulfamate or E2MATE) and/or its main metabolite, a steroid sulfatase inhibitor according to the invention (Estrone Sulfamate or E1 MATE) passed the placental barrier reversibly which supports a local availability of the steroid sulfatase inhibitor according to the invention for an effective activity in the prevention/treatment of premature uterine contractions.

### Example 2: Developmental toxicity in the rat

The safety of a use of a steroid sulfatase inhibitor according to the invention, measured by no or minor effects induced on foetus development during gestation in pregnant rat can be investigated as follows.

The effects of a steroid sulfatase inhibitor according to the invention (Estradiol Sulfamate or E2MATE) on the embryonic and foetal development of the rat was investigated when administered during the period of organogenesis (Days 6 to 17 of gestation). Four groups each of 20 time-mated female Sprague-Dawley rats were dosed with 0 (Control), 7.5, 22 or 60 µg/kg/day of Estardiol sulfamate from Day 6 to Day 17 of gestation. A further group of twenty females was dosed with ethinylestradiol at 80 µg/kg/day and acted as a positive control.

Clinical observations, bodyweight and food consumption were recorded and the progress and outcome of pregnancy was assessed on Day 20 of gestation. Foetal pathology to assess abnormalities and variations was carried out on all foetuses.

A No Observed Effect Level (NOEL) of Estradiol sulfamate for female toxicity was considered to be about 7.5 µg /kg/day. Observed female toxicity at the higher doses was limited to reduced food consumption and an associated reduction in bodyweight gain during gestation

At about 22 µg/kg/day PGL2001 and about 80 mcg/kg/day Ethinylestradiol (positive control) there were some minor effects in foetuses but no teratogenic action.

A No Observed Adverse Effect Level (NOAEL) of estradiol sulfamate for foetal development was therefore considered to be about 22 µg /kg/day.

### Example 3: Developmental Toxicity in the rabbit

The safety of a use of a steroid sulfatase inhibitor according to the invention, measured by no or minor effects induced on foetus development during gestation in pregnant rabbit can be investigated as follows.

The effects of a steroid sulfatase inhibitor according to the invention (Estradiol Sulfamate or E2MATE) on the embryonic and foetal development of the rabbit when administered during the period of organogenesis (Days 6 to 18 of gestation). Four groups each of between 20 to 25 time-mated female New Zealand White rabbits were dosed with 0 (Control), 5, 15 or 50 µg/kg/day from Day 6 to Day 18 of gestation. A further group of twenty females was administered with ethinylestradiol at 50 µg/kg/day and acted as a positive control. Clinical observations, bodyweight and food consumption were recorded and the progress and outcome of pregnancy was assessed on Day 28 of gestation. Foetal pathology to assess abnormalities and variations was carried out on all foetuses

The No Observed Adverse Effect Level for maternal toxicity during the period of organogenesis was considered to be about 5 µg/kg/day. Dosage-related maternal effects at higher doses were characterised by- and limited to lower bodyweight gain and food consumption. Estradiol sulfamate was generally better tolerated by the females on this study than ethinylestradiol (positive control). The No Observed Adverse Effect Level (NOAEL) for embryofoetal development following treatment with E2MATE was therefore considered to be about 15 µg/kg/day.

### Example 4: Inhibition of OT-induced uterine contractions

Steroid sulfatase inhibitors according to the invention may be tested for their activity in the inhibition and/or reduction of pre-term labour in the assay below, notably to establish the onset/duration of action and effective plasma concentrations affecting the blockade of OT-induced uterine contractions in pregnant rhesus monkeys

### Pharmacokinetics & Pharmacodynamics in Pregnant Monkeys

3 pregnant rhesus monkeys are surgically instrumented in jacket (at about 55 dGA) and catheter protection device (at about 70 dGA). Foetal hemodynamics is recorded by Doppler Ultrasonography at about 75-79 dGA and during the all length of the study such as at about 86 dGA, about 113-115 dGA, about129-131 dGA, about141-143 dGA, about 157-159 dGA and about 165-175 dGA: Ketamine injection (10-20 mg, i.v.) is used for light sedation during oral dosing, followed by 100 mg IM injection for standard ultrasound procedure (45 min).

At about 80 dGA an oral single dose of a steroid sulfatase inhibitor according to the invention (Estradiol Sulfamate or E2MATE) at a dosage of about 0.2- or 1- or 4 mg/kg is administered. A wash-out period of a minimum of 14 days is observed.

At about 100 dGA oral dosing of Estradiol Sulfamate is then repeated twice per week (about every 4 days) until about 160 dGA (e.g. 156 dGA). Frequent blood sampling over a period of 5 days following single dose administration, 1^{st} and last days of repeated administration is performed (or longer based on t_{1/2}). Blood sampling (3 mL), split between analysis of Estrone Sulfamate / Estrone Sulfamate whole blood exposure (whole blood collected in EDTA coated tubes) and dosage of STS activity in PBMCs, and steroid panel are carried out. Periodic non-invasive foetal monitoring is carried out (i.e. cardiovascular hemodynamics and growth measurements by Doppler ultrasound). Periodic cervical exams/swabs are performed during all the study. C-section (cesarean) delivery is carried out at 160 dGA (to collect blood, amniotic fluid and tissues).

The following parameters are measured:
- Maternal levels of Estradiol Sulfamate and its main active metabolite Estrone Sulfamate (foetal levels at C-section) in whole blood by LC-MS/MS;
- STS inhibitory activity in maternal PBMCs (Foetal levels at C-section) measured by radioassay measurement (Liquid scintillation counting in dual mode (³H/¹⁴C) as follows: the conversion of estrone sulphate to estrone by STS was measured by liquid scintillation counting. STS activity was related to the number of cells present and to the protein content of the sample. Cells were counted by a fluorescence assay correlating DNA signal to cell number and the protein content was determined by the Bradford method (*Bradford, 1976, Anal. Biochem., 72, 248*)*.* The STS activity was evaluated by adding tritium labelled estrone sulphate to the sample. After incubation, the converted estrone (still radiolabelled) was extracted by the organic scintillation cocktail while the remaining estrone sulphate stayed in the aqueous sample. ¹⁴C-estrone was added before extraction as internal standard. The estrone activity was measured by liquid scintillation.
- Maternal Steroid panel such as androstenedione, DHEA-S, DHEA, estradiol, estrone, progesterone, cortisol (foetal & amniotic fluid at C-section) by Elisa, LC-MS/MS or radioimmunoassay depending on the hormone;
- Placental tissue analysis for STS activity and Estradiol Sulfamate and its main active metabolite Estrone Sulfamate levels (at C-section) by radioassay measurement and LC-MS/MS, respectively;
- Foetal tissue (such as liver) & gestational tissue analysis for STS activity Estradiol Sulfamate and its main active metabolite Estrone Sulfamate levels (at C-section).

### Pharmacokinetics & Oxytocin Sensitivity in Pregnant Monkeys

The same experiment protocol as described above is carried out where an oxytocin (OT) sensitivity challenge is performed at about 75-79 dGA with incremental doses of OT achieved by increasing the infusion rate of OT stock dilution (10 mU/ml): OT infusion at 2 mU/kg/h, 2 ml/h infusion rate (30 min); OT infusion at 4 mU/kg/h, 4 ml/h infusion rate (30 min); OT infusion at 8 mU/kg/h, 8 ml/h infusion rate (30 min); OT infusion at 16 mU/kg/h, 16 ml/h infusion rate (30 min); OT infusion at 32 mU/kg/h, 32 ml/h infusion rate (30 min); OT infusion at 64 mU/kg/h, 64 ml/h infusion rate (30 min).

At about 80 dGA, an oral single dose of Estradiol Sulfamate at about 1 mg/kg is administered. A wash-out period of minimum 14 days is observed.

At about 95 dGA, an oxytocin (OT) sensitivity challenge is performed as described above. At about 100 dGA, an oral single dose of Estradiol Sulfamate at I mg/kg is administered. A wash-out period of minimum 14 days is observed. This protocol (OT challenge followed by the administration of Estradiol Sulfamate about 6 days after the OT challenge and a wash-out period of about 14 days) is repeated until term.

The same parameters as those described in Example 1 are followed.

## Claims

1. Use of a steroid sulfatase inhibitor, as well as pharmaceutically acceptable salts or a pharmaceutically active derivative thereof, for the preparation of a pharmaceutical composition for the treatment or prophylaxis of a disease or condition associated with premature uterine contractions.

2. A use according to claim 1 wherein the steroid sulfatase inhibitor is a sulfamate.

3. A use according to claims 1 or 2 wherein the steroid sulfatase inhibitor is a sulfamate of Formula (I): 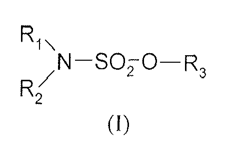 wherein R¹ and R² are independently selected from H, C₁-C₆ alkyl, C₂-C₆ alkenyl, C₂-C₆ alkynyl, alkoxy, C₃-C₈-cycloalkyl, heterocycloalkyl, acyl, aryl, heteroaryl, C₁-C₆ alkyl aryl, C₁-C₆ alkyl heteroaryl, C₁-C₆ alkyl C₃-C₈-cycloalkyl, C₁-C₆ alkyl heterocycloalkyl, aryl C₁-C₆ alkyl, heteroaryl C₁-C₆ alkyl, C₃-C₈-cycloalkyl C₁-C₆ alkyl and heterocycloalkyl C₁-C₆ alkyl or R¹ and R² form together an alkenyl group; R³ is a group selected from a group of Formulae (II), (III) and (IV): 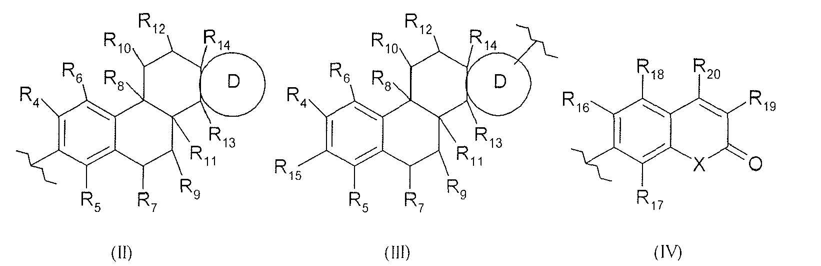 wherein R⁴ and R⁵ are independently selected from H; halogen; nitro; amino; sulfanyl; acyl; thioalkyl; C₁-C₆ alkyl; C₂-C₆ alkenyl; alkoxy; alkoxy alkyl, amino alkyl; C₃-C₈-cycloalkyl; and aryl; R⁶ R⁷, R⁸, R⁹, R¹⁰, R¹¹, R¹², R¹³ and R¹⁴ are independently selected from H; OH; halogen and C₁-C₆ alkyl; R¹⁵ is selected from selected from H; OH; halogen; sulfamate and C₁-C₆ alkyl; R¹⁶, R¹⁷ and R¹⁸ are independently selected from H; OH; halogen; C₁-C₆ alkyl; C₂-C₆ alkenyl; alkoxy; acylamino; amino and sulfonyl; R¹⁹ and R²⁰ are independently selected from H; OH; halogen; C₁-C₆ alkyl; C₂-C₆ alkenyl; alkoxy; acylamino; amino and sulfonyl; or R¹⁹ and R²⁰ form together a C₃-C₁₄-cycloalkyl ring; R²¹ is selected from H and C₁-C₆ alkyl; R²² and R²³ are independently selected from H; OH; halogen; C₁-C₆ alkyl; D is a ring selected from C₃-C₈-cycloalkyl and heterocycloalkyl; X is selected from O, NR²¹ and CR²²R²³.

4. A use according to any one of claims 1 to 3 wherein the steroid sulfatase inhibitor is a sulfamate of Formula (I) wherein R¹ and R² are independently selected from H and C₁-C₆ alkyl.

5. A use according to any one of claims 1 to 4 wherein the steroid sulfatase inhibitor is a sulfamate of Formula (I) wherein at least one of R¹ and R² is H.

6. A use according to any one of claims 1 to 5 wherein the steroid sulfatase inhibitor is a sulfamate of Formula (I) wherein R³ is of Formula (II).

7. A use according to any one of claims 1 to 6 wherein the steroid sulfatase inhibitor is a sulfamate of Formula (I) wherein R³ is of Formula (II'): 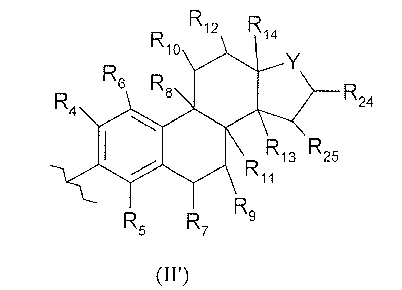 wherein R⁴, R⁵, R⁶, R⁷, R⁸, R⁹, R¹⁰, R¹¹, R¹², R¹³ and R¹⁴ are as defined in any one of the preceding claims; R²⁴ and R²⁵ are independently selected from H; OH; halogen; C₁-C₆ alkyl; C₂-C₆ alkenyl; alkoxy; acylamino; amino and sulfonyl; Y is selected from acyl; alkoxy; -C(O)-; C₁-C₆ alkyl; C₂-C₆ alkenyl; C₂-C₆ alkynyl; C=N-OR²⁹ and CR²⁶R²⁷; R²⁶ and R²⁷ are independently selected from H; OH; nitrile; sulfamate; alkoxy; carbonyl; amino; nitrile; C₁-C₆ alkyl; C₂-C₆ alkenyl; aryl; heteroaryl; aryl C₁-C₆ alkyl; aminocarbonyl; acylamino; alkoxycarbonyl; and sulfonyloxy; R²⁹ is selected from H; C₁-C₆ alkyl and carbonyl.

8. A use according to any one of claims 1 to 6 wherein the steroid sulfatase inhibitor is a sulfamate of Formula (I) wherein R³ is of Formula (II"): 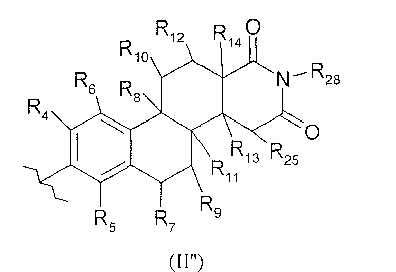 wherein R⁴, R⁵, R⁶, R⁷, R⁸, R⁹, R¹⁰, R¹¹, R¹², R¹³, R¹⁴ and R²⁵ are as defined in any of the preceding claims; R²⁸ is selected from H; OH; C₁-C₆ alkyl; C₂-C₆ alkenyl; aryl C₁-C₆ alkyl; heteroaryl C₁-C₆ alkyl; optionally substituted C₃-C₈-cycloalkyl C₁-C₆ alkyl such as optionally substituted cyclopropyl methyl; optionally substituted aryl; optionally substituted heteroaryl; optionally substituted C₃-C₈-cycloalkyl; and optionally substituted heterocycloalkyl.

9. A use according to any one of claims 1 to 6 wherein the steroid sulfatase inhibitor is a sulfamate of Formula (I) wherein R³ is of Formula (II"'): 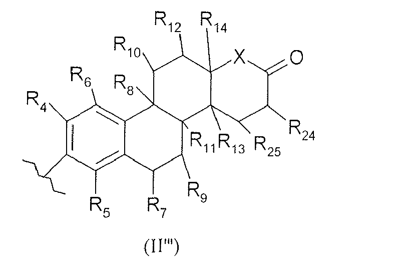 wherein R⁴, R⁵, R⁶, R⁷, R⁸, R⁹, R¹⁰, R¹¹, R¹², R¹³, R¹⁴, R²⁴ and R²⁵ are as defined in any of the preceding claims; X is selected from N and O.

10. A use according to any one of claims 1 to 5 wherein the steroid sulfatase inhibitor is a sulfamate of Formula (I) wherein R³ is of Formula (III).

11. A use according to claim 10 wherein R³ is of Formula (III'): 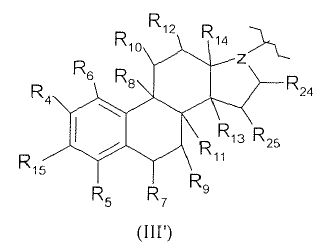 wherein R⁴, R⁵, R⁶, R⁷, R⁸, R⁹, R¹⁰, R¹¹, R¹², R¹³, R¹⁴, R¹⁵, R²⁴ and R²⁵ are as defined in any of the preceding claims; Z is CR³⁰; R³⁰ is selected from H and C₁-C₆ alkyl.

12. A use according to any one of claims 1 to 11 wherein the steroid sulfatase inhibitor is a sulfamate of Formula (I) wherein R⁶, R⁷, R⁸, R⁹, R¹⁰, R¹¹, R¹² and R¹³ are H.

13. A use according to any one of claims 1 to 12 wherein the steroid sulfatase inhibitor is a sulfamate of Formula (I) wherein R¹⁴ is C₁-C₆ alkyl.

14. A use according to any one of claims 1 to 5 wherein the steroid sulfatase inhibitor is a sulfamate of Formula (I) wherein R³ is of Formula (IV).

15. A use according to claim 14 wherein R³ is of Formula (IV'): 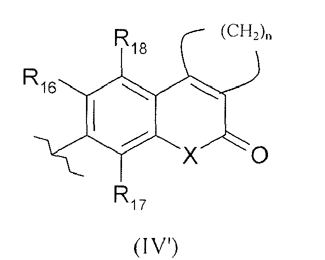 wherein R¹⁶, R¹⁷ and R¹⁸ are as defined in any of the preceding claims; n is an integer selected from 3 to 14.
